# EUROPEAN PATENT SPECIFICATION

(11) **EP 4 232 125 B1**
(45) Date of publication and mention of the grant of the patent: **18.02.2026**
(21) Application number: 21790223.8
(22) Date of filing: 19.10.2021
(51) Int. Cl.: A61M 5/32, A61M 5/50, A61M 5/20

(54) **AN ASSEMBLY FOR A MEDICAMENT DELIVERY DEVICE**
ANORDNUNG FÜR EINE MEDIKAMENTENABGABEVORRICHTUNG
ENSEMBLE POUR DISPOSITIF D'ADMINISTRATION DE MÉDICAMENTS

(30) Priority: 21.10.2020 EP 20202950
(43) Date of publication of application: 30.08.2023
(73) Proprietor: SHL Medical AG, 6302 Zug (CH)
(72) Inventor: PÅLMAN, Fredrik, 11548 Stockholm (SE); CARLSSON, Daniel, 122 43 Enskede (SE)
(86) International application number: PCT/EP2021/078873
(87) International publication number: WO 2022/084276

(56) References cited:
- WO-A1-2017/144203
- WO-A1-2020/141043
- US-A1- 2018 221 589
- US-B1- 6 830 560

## Description

### TECHNICAL FIELD

The present disclosure generally relates to medicament delivery devices.

### BACKGROUND

Medicament delivery devices such as injectors may have a safety shield which extends from the device body. The usually spring-biased safety shield may be axially movable relative to the device body. When a user is to administer medication, the device is pressed towards the site of administration. The safety shield is then moved into the device body activating a triggering mechanism to expel medicament. Moreover, in the case of injectors, the needle is exposed and penetrates the skin.

Medicament delivery devices may be provided with a cap. The cap is fitted to the front end of the device to prevent the safety shield from moving into the body by accident and expose the delivery member and/or activate the device. If the medicament delivery device is accidentally dropped on the floor, there is a risk that the safety shield moves into the body and activates the device. This risk can be reduced by designing a device with a delivery member cover with an extended stroke length and/or selecting a spring with higher spring constant. These design modifications however make the device less user-friendly. Some examples can be found in WO2020/141043, US2018/221589, WO2017/144203A1, and US6830560B1.

### SUMMARY

The invention is defined by the appended claims. An object of the present disclosure is to provide an assembly for a medicament delivery device which solves, or at least mitigates problems of the prior art.

There is according to a first aspect of the present disclosure provided an assembly for a medicament delivery device, the assembly comprising: a body, a delivery member cover arranged in the body and configured to move axially relative to the body from an extend position relative to the body to a retracted position, a rotator configured to be rotatable relative to the body, and a cap configured to receive a portion of the rotator when the delivery member cover is in the extended position, and wherein the cap is configured to be removably attached to and rotationally locked relative to the body, wherein the rotator has a first locking structure and the cap has a second locking structure configured to engage with the first locking structure when the delivery member cover is in the extended position, to prevent rotation of the rotator relative to the cap in a first rotational direction and thereby maintain the rotator in a first rotational position relative to the body, wherein the delivery member cover has a first blocking structure configured to engage with a second blocking structure, which is axially fixed relative to the body, when the rotator is in the first rotational position to prevent the delivery member cover to move axially from the extended position towards the retracted position, wherein one of the first blocking structure and the second blocking structure has a cam surface configured to cooperate with the other one of the first blocking structure and the second blocking structure such that when the cap has been removed from the body and the delivery member cover is subjected to an axial force directed distally, the rotator is rotated in the first rotational direction, causing the first blocking structure and the second blocking structure to disengage, enabling the delivery member cover to move axially from the extended position towards the retracted position.

Thus, when the cap is fitted to the body the delivery member cover is prevented from axial movement. Once the cap has been removed, the rotator is free to rotate in the first direction, whereby the delivery member cover can be disengaged from the body. The delivery member cover is thereby able to move from the extended position towards the retracted position.

An effect obtainable thereby is that the risk that the delivery member cover moves towards the retracted position in case that a medicament delivery device comprising the assembly is subjected to a sudden impact, for example by being dropped, is reduced.

As used herein, the term "proximal end" of a component, assembly or the medicament delivery device, is an end which is facing the site of medical expulsion when the assembly is in use. The "distal end" is the opposite end relative to the proximal end. "Proximal direction" means a direction from the distal end towards the proximal end. "Distal direction" means a direction from the proximal end towards the distal end. The term "distally" is a relative term that means that a feature is located closer to the distal end of a component, the assembly, or of a medicament delivery device comprising the assembly than another feature. The term "proximally" means opposite to "distally".

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", "circumferentially", "rotation", "rotational" and "rotationally" refer to a direction generally perpendicular to the longitudinal direction and at least partially extending around the longitudinal direction.

According to one embodiment the delivery member cover is the rotator, wherein the body comprises the second blocking structure, and wherein the cap is configured to receive a proximal portion of the delivery member cover when the delivery member is in the extended position.

According to one embodiment one of the first locking structure and the second locking structure comprises a recess and the other one of the first locking structure and the second locking structure comprises a protrusion configured to be received into the recess to prevent rotation of the delivery member cover in the first rotational direction.

According to one embodiment the recess is arranged in a radial plane of the delivery member cover.

According to one embodiment the recess extends in a circumferential direction of the delivery member cover.

According to one embodiment the protrusion extends in an axial direction of the cap.

According to one embodiment first locking structure comprises the recess and the second locking structure comprises the protrusion.

According to one embodiment not according to the invention the rotator is a separate component from the
delivery member cover, the rotator being provided with the second blocking structure, and wherein the rotator is arranged concentrically with the delivery member cover radially outside of the delivery member cover. The rotator is thus according to this embodiment different from the delivery member cover. The rotator may in this case be axially fixed relative to the body.

According to one embodiment the first blocking structure comprises a first radial protrusion, and the second blocking structure comprises a second radial protrusion configured to be arranged axially aligned with and distally relative to the first radial protrusion in the first rotational position of the delivery member cover to prevent the delivery member cover to move towards the retracted position.

According to one embodiment the delivery member cover comprises a radially extending first ramp structure that tapers in a direction from the proximal portion towards a distal end of the delivery member cover, wherein the first ramp structure is arranged offset in the first rotational direction relative to the first radial protrusion and closer to the proximal portion than the first radial protrusion, and wherein the second radial protrusion is configured to move over the first ramp structure when the delivery member cover is moved towards the retracted position.

According to one embodiment the first ramp structure has a proximal end portion provided with a radial guide surface extending along a helical direction of the delivery member cover, the radial guide surface being configured to guide the second radial protrusion when the delivery member cover is moved towards the extended position, causing rotation of the delivery member cover in a second rotational direction opposite to the first rotational direction relative to the body.

According to one embodiment the delivery member cover has a second ramp structure which in the circumferential direction is arranged adjacent to the first ramp structure, wherein the radial guide surface is configured to guide the second radial protrusion towards the second ramp structure when the delivery member cover is moved from the retracted position towards the extended position, wherein the second ramp structure is tapering in an opposite direction relative to the tapering of the first ramp structure.

According to one embodiment the second ramp structure has a snap-lock functionality configured to prevent the delivery member cover to move towards the retracted position when the second radial protrusion has moved axially past the second ramp structure.

According to one embodiment the delivery member cover comprises a radial structure axially aligned with and arranged distally relative to the second ramp structure, the radial structure being configured to prevent the delivery member cover to move further proximally when the second radial protrusion has moved past the second ramp structure.

There is according to a second aspect provided a medicament delivery device comprising an assembly according to the first aspect.

There is according to a third aspect provided an assembly for a medicament delivery device, the assembly comprising: a body, a delivery member cover arranged in the body and configured to move axially relative to the body from an extend position relative to the body to a retracted position, and wherein the delivery member cover is configured to be rotatable relative to the body, and a cap configured to receive a proximal portion of the delivery member cover extending from the body when the delivery member cover is in the extended position, and wherein the cap is configured to be removably attached to and rotationally locked relative to the body, wherein the delivery member cover has a first locking structure and the cap has a second locking structure configured to engage with the first locking structure when the delivery member cover is in the extended position, to prevent rotation of the delivery member cover relative to the cap in a first rotational direction and thereby maintain the delivery member cover in a first rotational position relative to the body, wherein the delivery member cover has a first blocking structure and the body has a second blocking structure configured to engage with the first blocking structure when the delivery member cover is in the first rotational position to prevent the delivery member cover to move axially from the extended position towards the retracted position, wherein one of the first blocking structure and the second blocking structure has a cam surface configured to cooperate with the other one of the first blocking structure and the second blocking structure such that when the cap has been removed from the body and the delivery member cover is subjected to an axial force directed distally, the delivery member cover is rotated in the first rotational direction, causing the first blocking structure and the second blocking structure to disengage, enabling the delivery member cover to move axially from the extended position towards the retracted position.

Thus, when the cap is fitted to the body the delivery member cover is prevented from axial movement because the delivery member cover is not allowed to rotate in the first direction. Once the cap has been removed, the delivery member cover is free to rotate in the first direction, whereby the delivery member cover can be disengaged from the body. The delivery member cover is thereby able to move from the extended position towards the retracted position.

In this example, the delivery member cover can be rotated during the assembling process via the axial movement of the cap by the cam surface of the one of the first blocking structure and the second blocking structure has a cam surface and the other one of the first blocking structure and the second blocking structure.

In a preferred example, the delivery member cover comprises a spinning section at the proximal end of the delivery member cover. The spinning section is rotatable relative to the delivery member cover and is configured to contact the medicament delivery site during the medicament delivery operation. Thereby, an unintentional rotation of the delivery member cover during the medicament delivery operation can be prevented.

An effect obtainable thereby is that the risk that the delivery member cover moves towards the retracted position in case that a medicament delivery device comprising the assembly is subjected to a sudden impact, for example by being dropped, is reduced.

The first blocking structure may be provided with the cam surface.

According to one embodiment one of the first locking structure and the second locking structure comprises a recess and the other one of the first locking structure and the second locking structure comprises a protrusion configured to be received into the recess to prevent rotation of the delivery member cover in the first rotational direction.

According to one embodiment the recess is arranged in a radial plane of the delivery member cover.

According to one embodiment the recess extends in a circumferential direction of the delivery member cover.

According to one embodiment the protrusion extends in an axial direction of the cap.

According to one embodiment the first locking structure comprises the recess and the second locking structure comprises the protrusion.

Alternatively, the first locking structure comprises the protrusion and the second locking structure comprises the recess.

According to one embodiment the first blocking structure comprises a first radial protrusion, and the second blocking structure comprises a second radial protrusion configured to be arranged axially aligned with and distally relative to the first radial protrusion in the first rotational position of the delivery member cover to prevent the delivery member cover to move towards the retracted position.

According to one embodiment the delivery member cover comprises a radially extending first ramp structure that tapers in a direction from the proximal portion towards a distal end of the delivery member cover, wherein the first ramp structure is arranged offset in the first rotational direction relative to the first radial protrusion and closer to the proximal portion than the first radial protrusion, and wherein the second radial protrusion is configured to move over the first ramp structure when the delivery member cover is moved towards the retracted position.

According to one embodiment the first ramp structure has a proximal end portion provided with a radial guide surface extending along a helical direction of the delivery member cover, the radial guide surface being configured to guide the second radial protrusion when the delivery member cover is moved from the retracted portion towards the extended position, causing rotation of the delivery member cover in a second rotational direction opposite to the first rotational direction relative to the body.

The radial guide surface is a second cam surface which translates linear movement of the delivery member cover as it moves towards the extended position to a rotational movement of the delivery member cover in the second rotational direction.

According to one embodiment the delivery member cover has a second ramp structure which in the circumferential direction is arranged adjacent to the first ramp structure, wherein the radial guide surface is configured to guide the second radial protrusion towards the second ramp structure when the delivery member cover is moved towards the extended position, wherein the second ramp structure is tapering in an opposite direction relative to the tapering of the first ramp structure.

According to one embodiment the second ramp structure has a snap-lock functionality configured to prevent the delivery member cover to move towards the retracted position when the second radial protrusion has moved axially past the second ramp structure.

According to one embodiment the delivery member cover comprises a radial structure axially aligned with and arranged distally relative to the second ramp structure, the radial structure being configured to prevent the delivery member cover to move further proximally when the second radial protrusion has moved past the second ramp structure.

One embodiment comprises a resilient member configured to urge the delivery member cover towards the extended position.

There is according to a fourth aspect of the present disclosure provided a medicament delivery device comprising an assembly according to the third aspect.

According to one embodiment the medicament delivery device is an injector, such as an auto-injector, or an inhaler.

Generally, all terms used in the claims are to be interpreted according to their ordinary meaning in the technical field, unless explicitly defined otherwise herein. All references to "a/an/the element, apparatus, component, means, etc." are to be interpreted openly as referring to at least one instance of the element, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 shows a perspective view of one example of a medicament delivery device;
Fig. 2 shows the medicament delivery device with an outer cap member removed from an inner cap member;
Fig. 3 is a perspective view of an example of a delivery member cover;
Fig. 4 shows a perspective view of a body;
Fig. 5a is a close-up view of the inner cap member fitted over the delivery member cover in Fig. 3;
Fig. 5b shows a cross-section along lines A-A in Fig. 5a;
Fig. 5c is a close-up view when the inner cap member has been partly removed from the delivery member cover; and
Fig. 6a-6h show the interaction between the delivery member cover and the body as the delivery member cover is moved from the extended position to the retracted position and back to the extended position.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

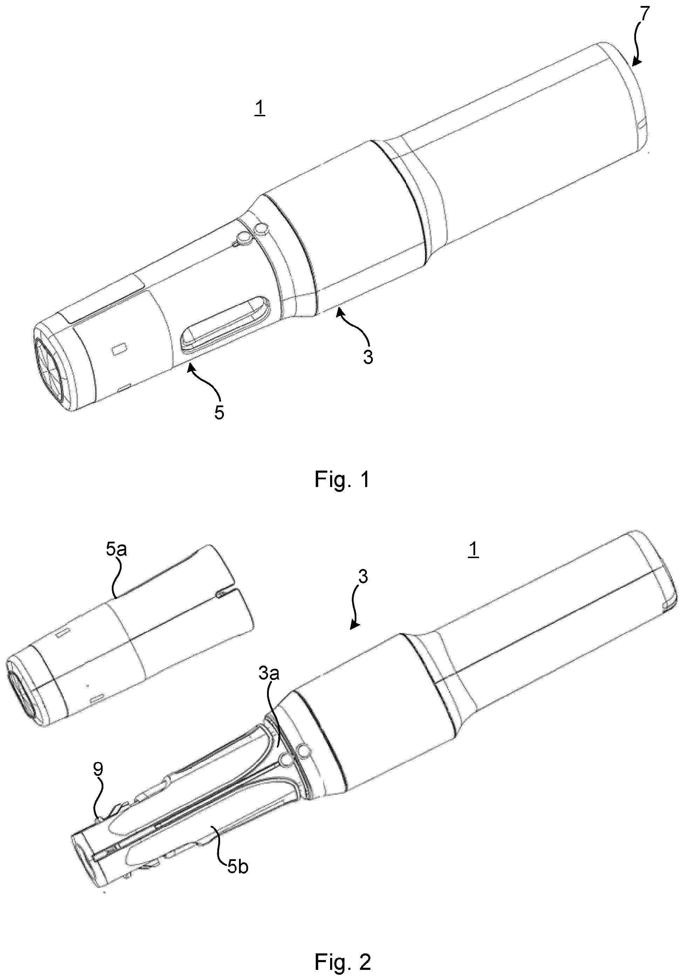

Fig. 1 depicts an example of a medicament delivery device 1. The exemplified medicament delivery device 1 is an injector. The injector may be an auto-injector. The medicament delivery device 1 could alternatively for example be an inhaler.

The medicament delivery device 1 has a housing 3 which may be formed by one or more parts.

The medicament delivery device 1 comprises a removable cap 5. The cap 5 is configured to be assembled with the housing 3. According to the present example the housing 3 has a proximal part or body (not shown in Fig. 1), which the cap 5 receives when the cap 5 is assembled with the housing 3. The cap 5 is configured to be rotationally locked relative to the housing 3.

The cap 5 forms a proximal end, or proximal end, of the medicament delivery device 1. The medicament delivery device 1 has a distal end 7. The distal end 7 is the distal end of the medicament delivery device 1.

Turning now to Fig. 2, the medicament delivery device 1 is shown with the cap 5 partly removed. According to the example, the cap 5 includes an outer cap member 5a and an inner cap member 5b. The outer cap member 5a is configured to be arranged concentrically with the inner cap member 5b. The outer cap member 5a is configured to be arranged radially outside of the inner cap member 5b. In Fig. 2, the outer cap member 5a has been removed from the inner cap member 5b. The inner cap member 5b is fitted to the body 3a. As mentioned previously, the body 3a is a proximal part of the housing 3.

Alternatively, the cap could be made as a single piece cap. In this case the inner cap member and the outer cap member are integral.

The medicament delivery device 1 comprises a delivery member cover 9. The delivery member cover 9 may be an elongated structure. The delivery member cover 9 may be a sleeve or tubular structure configured to be arranged around a delivery member and/or a medicament container.

According to the first example disclosed herein the medicament delivery cover is a rotator. This is because the medicament delivery cover is configured to be rotatable relative to the body 3a as will be described in more detail in the following.

The delivery member cover 9 is configured to be received by the body 3a. The body 3a has a proximal opening through which the delivery member cover 9 extends. The delivery member cover 9 is configured to extend from the proximal end of the body 3a.

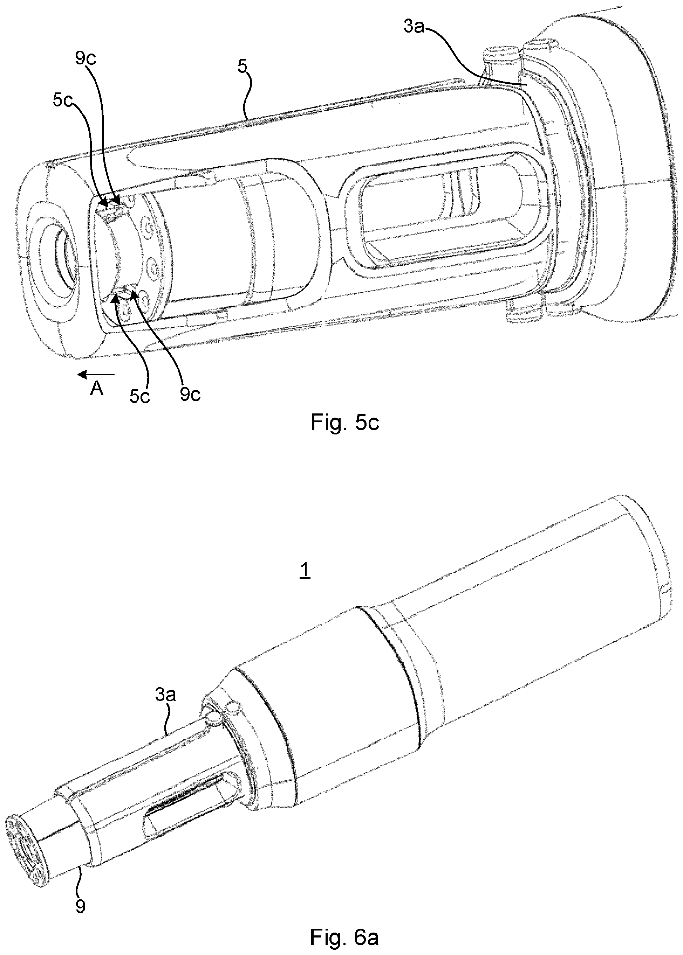

Fig. 6a provides a better view of the delivery member cover 9 extending from the body 3a. The delivery member cover 9 is configured to be axially movable relative to the body 3a between an extended position and a retracted position relative to the body 3a. The delivery member cover 9 is configured to move from the extended position to the retracted position, and back to the extended position.

The extended position is the default position of the delivery member cover 9. The medicament delivery device 1 may comprise a resilient member such as a spring, configured to urge the delivery member cover 9 towards the extended position. The delivery member cover 9 is configured to be biased towards the extended position.

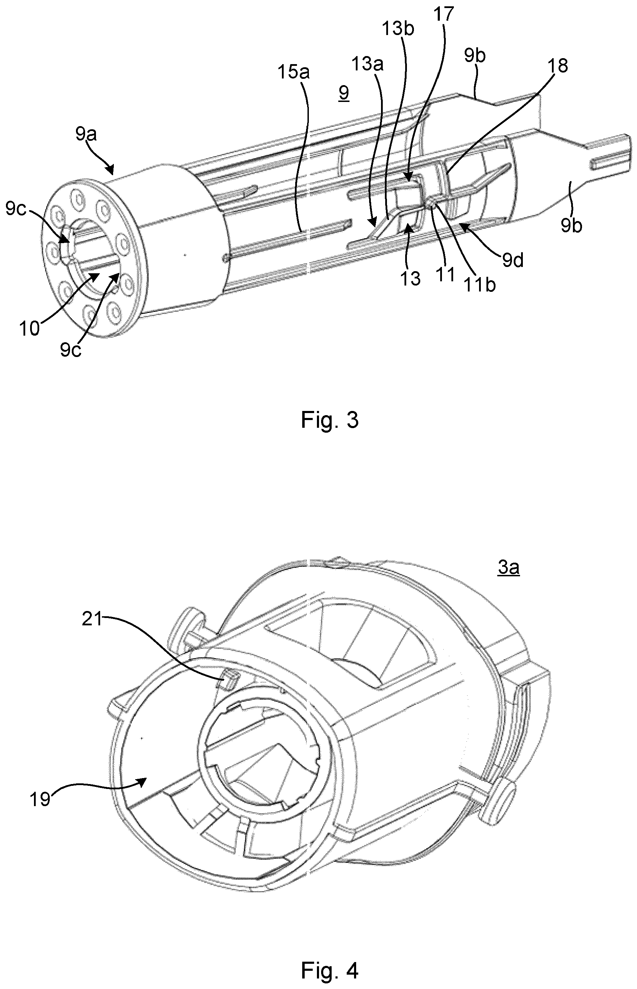

An example of the delivery member cover 9 will now be described with reference to Fig. 3.

The delivery member cover 9 has a proximal portion 9a. The proximal portion 9a may be tubular. The delivery member cover 9 has two legs 9b extending distally from the proximal portion 9a. The legs 9b hence extend distally from the proximal portion 9a.

The proximal portion 9a has an axially extending central through-opening 10 configured to receive a delivery member, such as a needle, of the medicament delivery device 1. The proximal portion 9a is provided with a first locking structure 9c. The first locking structure 9c comprises recesses. The recesses open from the through-opening 10. Each recess is arranged in a radial plane of the delivery member cover 9. Each recess is a radially extending recess. Each recess has an extension in the circumferential direction of the delivery member cover 9. The recesses may be arranged opposite to each other in the through-opening 10.

According to the example, each recess has an extension in the circumferential direction corresponding to less than 90 degrees, such as less than 60 degrees, such as less than 45 degrees, of the inner circumference of the through-opening 10.

Each leg 9b is provided with a respective first blocking structure 9d. The first blocking structures 9d are provided on a radially outer surface of the legs 9b. The first blocking structure 9d extends radially outwards.

The first blocking structure 9d shown in Fig. 3 comprises a first radial protrusion 11. According to the example shown in Fig. 3, the first radial protrusion 11 has a cam surface 11a. The cam surface 11a is at least partly directed towards the distal end of the delivery member cover 9.

The delivery member cover 9 comprises a radially extending first ramp structure 13 that tapers in a direction from the proximal portion 9a towards a distal end of the delivery member cover 9.

The first ramp structure 13 is arranged closer to the proximal portion 9a than the first radial protrusion 11.

The first ramp structure 13 is arranged offset in a first rotational direction relative to the first radial protrusion 11. The first ramp structure 13 is offset in the circumferential direction of the delivery member cover 9 relative to the first radial protrusion 11.

The first ramp structure 13 has a proximal end portion 13a provided with a radial guide surface 13b which extends radially outwards. The radial guide surface 13b is arranged proximally relative to the tapering portion of the first ramp structure 13. The radial guide surface 13b extends along a helical direction of the delivery member cover 9. The radial guide surface 13b hence intersects an axial plane extending through the delivery member cover 9. The radial guide surface 13b is a cam surface.

The delivery member cover 9 has an axial rib 15a which extends in the axial direction of the delivery member cover 9.

The delivery member cover 9 has a second ramp structure 17 is arranged adjacent to the first ramp structure 13 in the circumferential direction of the delivery member cover 9. The second ramp structure 17 is arranged adjacent to the first ramp structure 13 in a second rotational direction opposite to the first rotational direction.

The first ramp structure 13 and the second ramp structure 17 may be fully axially aligned. The first ramp structure 13 and the second ramp structure taper in opposite direction. The second ramp structure 17 is tapering in a direction from the distal end of the delivery member cover 9 towards the proximal portion 9a.

The radial guide surface 13b is configured to transition into the second ramp structure 17.

The delivery member cover 9 may comprise a radial structure 18 axially aligned with and arranged distally relative to the second ramp structure 17.

Fig. 4 shows a perspective view from the proximal of the body 3a. The body 3a has a proximal opening 19 which extends axially through the body 3a. The delivery member cover 9 is configured to be arranged in the body 3a and extends from the proximal opening 19 in the extended position.

The delivery member cover 9 is configured to be axially moved relative to the body 3a. The delivery member cover 9 is configured to be able to rotate relative to the body 3a.

The body 3a has a second blocking structure 21. The second blocking structure 21 may comprise a second radial protrusion. The second blocking structure 21 extends radially inwards. The second blocking structure 21 is configured to engage with the first blocking structure 9d when the delivery member cover 9 is in the first rotational position to prevent the delivery member cover 9 to move axially from the extended position towards the retracted position. The second blocking structure 21 is configured to cooperate with the first radial protrusion 11, with the first ramp structure 13 and subsequently with the second ramp structure 17 when the medicament delivery device 1 is being operated, as will be explained in more detail in the following.

In a preferred example, the first ramp structure comprises a slot for guiding the axial movement of the second blocking structure, so that the second blocking structure will not accidentally into a position that is axially lined up with the second ramp structure.

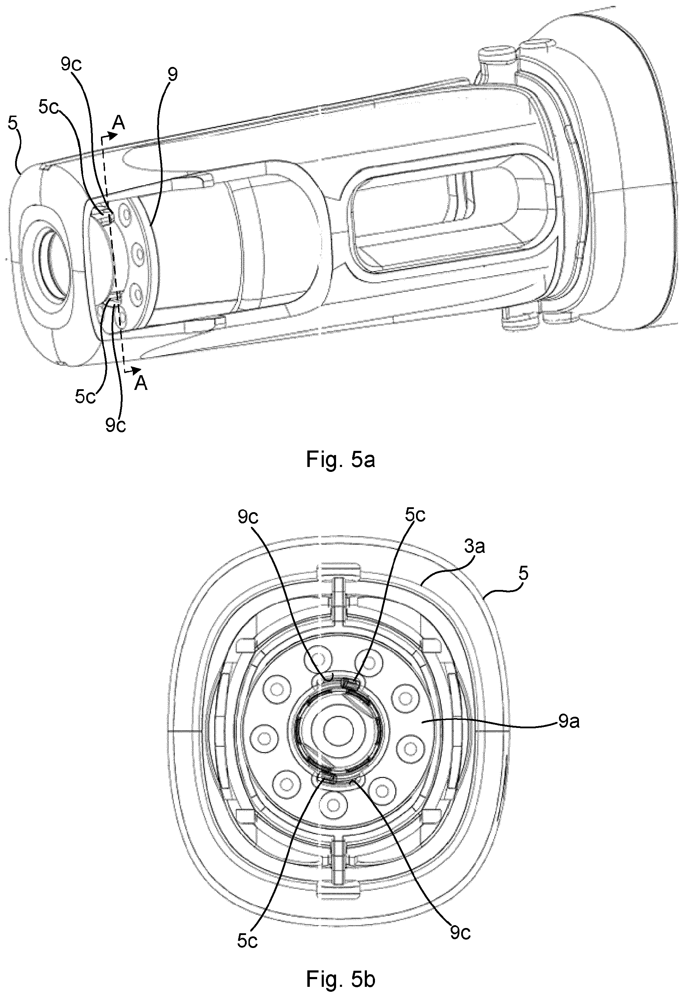

Similarly, the second ramp structure can also have a slot to prevent a transverse movement of the second blocking structure.Fig. 5a shows a longitudinal section of the cap 5 when mounted to the body 3a. The cap 5 has a second locking structure 5c configured to engage with the first locking structure 9c of the delivery member cover 9.

The second locking structure 5c is arranged inside the cap 5. The inner cap member 5b may be provided with the second locking structure 5c.

The second locking structure 5c comprises a protrusion configured to be received into the recess of the first locking structure 9c to prevent rotation of the delivery member cover 9 in the first rotational direction when the cap 5 is fitted to the body 3a. The protrusion may extend in the axial direction of the cap 5.

The second locking structure 5c may comprise several protrusions, each being configured to be received in a respective recess of the delivery member cover 9.

The protrusion bears against at least one circumferential end surface of the recess when the cap is fitted to the body 3a. Thus, the delivery member cover 9 is prevented from rotation in a first rotational direction when the cap 5 is fitted to the body 3a.

Fig. 5b shows a cross-section along lines A-A in Fig. 5a. In Fig. 5b it can be seen that the protrusions 5c are arranged in a respective one of the recesses of the delivery member cover 9.

The operation of the delivery member device 1 will be described with reference to Figs 5c to 6h.

In Fig. 5c the cap 5 is being pulled off the body 3a. The cap 5 is thus pulled in the proximal direction as shown by arrow A. The protrusions of the second locking structure 5c are thus moved axially in the forward or proximal direction and moved out from the recesses of the first locking structure 9c. The first locking structure 9c and the second locking structure 5c are thus disengaged. The delivery member cover 9 is thereby able to rotate in the first rotational direction.

Fig. 6a shows the medicament delivery device 1 when the cap 5 has been removed from the body 3a. The delivery member cover 9 is in the extended position relative to the body 3a.

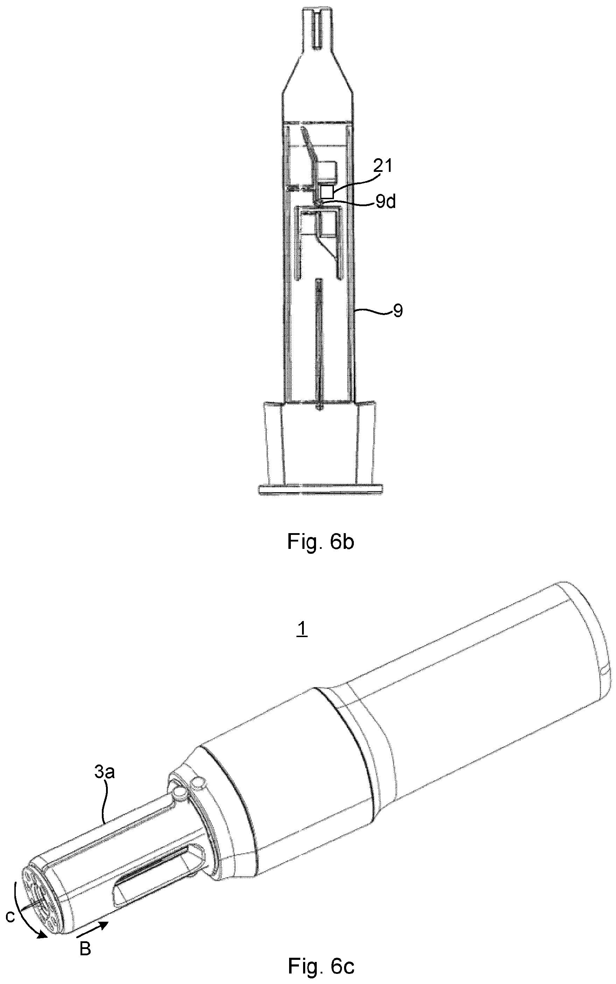

Fig. 6b illustrates the interaction between the delivery member cover 9 and the second blocking structure 21 of the body 3a when the medicament delivery device 1 is in the extended position as shown by Fig. 6a. The delivery member cover 9 is in the first rotational position. The second blocking structure 21 bears against the first blocking structure 9d of the delivery member cover 9. The second blocking structure 21 bears against the cam surface 11a of the first radial protrusion 11. The second blocking structure 21 is arranged distally relative to the first radial protrusion 11 such that the delivery member cover 9 cannot be moved distally from the extended position towards the retracted position without prior rotation of the delivery member cover 9. However, as can be understood from the foregoing, the delivery member cover 9 is able to rotate in the first rotational direction from the first rotational position when the cap 5 has been removed from the body 3a.

In Fig. 6c, the delivery member cover 9 has been moved distally into the body 3a to the retracted position, as shown by the arrow B. For an injector, this is typically done by pressing the delivery member cover 9 against the site of injection. The delivery member cover 9 is slightly rotated in the first rotational direction C.

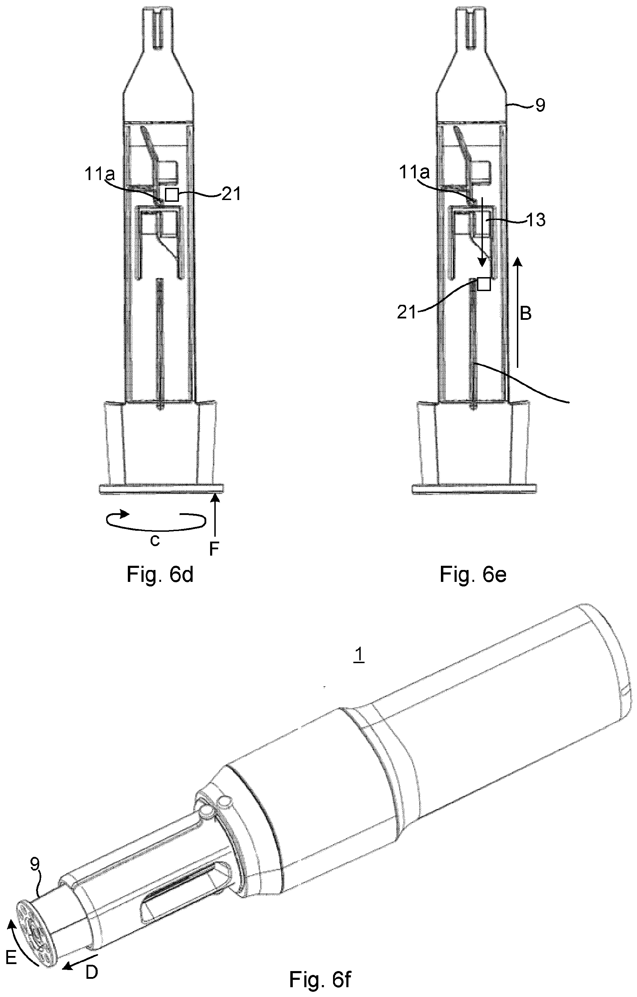

Fig. 6d shows that the delivery member cover 9 is being rotated in the first rotational direction C as the delivery member cover 9 is subjected to a distally directed force F. Due to the cam surface 11a, the axial force is transformed into a rotation of the delivery member cover 9 relative to the body 3a. The delivery member cover 9 is thus rotated in the first rotational direction C.

When the delivery member cover 9 has been rotated in the first rotational direction C the second blocking structure 21 is disengaged from the first blocking structure 9d. The delivery member cover 9 is thereby able to be moved further into the body 3a towards the retracted position.

The axial rib 15a acta as guides for the second blocking structure 21 to ensure that the delivery member cover 9 and the body 3a are aligned as required when the delivery member cover 9 is returned to the extended position.

Fig. 6e depicts how the second blocking structure 21 is moved axially over the first ramp structure 13 as the delivery member cover 9 is moved towards the retracted position. The first ramp structure 13 may be configured to flex radially inwards to enable the second blocking structure 21 to move past it.

As the delivery member cover 9 is moved towards the retracted position activation of the medicament delivery device 1 is initiated. The activation may be carried out in a plurality of ways and will not be described in any further detail herein.

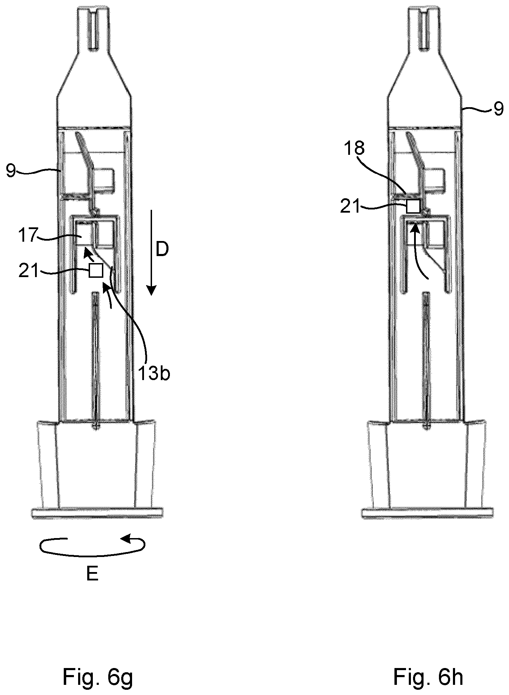

Fig. 6f shows the medicament delivery device 1 when the delivery member cover 9 returns to the extended position from the retracted position, as shown by the arrow D. The delivery member cover 9 is biased towards the extended position, so that when the user removes the medicament delivery device 1 from the site of expulsion, the delivery member cover 9 is returned to the extended position. The delivery member cover 9 is rotated slightly in the second rotational direction E, opposite to the first rotational direction C. This is illustrated in Fig. 6g, where the delivery member cover 9 moves towards the extended position and the second blocking structure 21 is moved towards the radial guide surface 13b, or cam surface, of the first ramp structure 13. The second blocking structure 21 is thereby guided towards the second ramp structure 17. This causes a rotation of the delivery member cover 9 in the second rotational direction E. The second blocking structure 21 is subsequently moved over the second ramp structure 17, which may me flexible radially inwards. The second ramp structure 17 may have a snap-lock functionality. Once the second blocking structure 21 has moved past the second ramp structure 17, the delivery member cover 9 is prevented from movement towards the retracted position, as shown in Fig. 6h.

The radial structure 18 may be arranged distally relative to the second blocking structure 21 when the second blocking structure 21 has moved past the second ramp structure 17. The radial structure 18 prevents the delivery member cover 9 from being pulled out from the body 3a, because it acts as a radial barrier that bears against the second blocking structure 21.

According to another example not according to the invention of the medicament delivery device, the
medicament delivery device may additionally comprise a rotator which is separate from the delivery member cover. In this case, the delivery member cover is configured to be rotationally locked relative to the body.

The rotator is arranged concentrically with the delivery member cover, in between the delivery member cover and the body. The rotator is arranged rotatably relative to the body. The rotator may be prevented from moving axially distally relative to the body. The rotator and the body may thus be engaged to prevent rearward movement of the rotator.

The delivery member cover is provided with the first blocking structure just like in the first example. The rotator is provided with the second blocking structure. The second blocking structure may be provided on the inner surface of the rotator.

The rotator is in this example provided with the first locking structure, configured to engage with the second locking structure of the cap. Thus, when the cap is fitted to the body, the rotator is prevented from rotation in the first rotational direction. The delivery member cover is therefore prevented to move from the extended position towards the retracted position. When the cap has been removed, the rotator is allowed to rotate in the first rotational direction.

When the cap has been removed, axial force in the distal direction on the delivery member cover causes interaction between the first blocking structure and the second blocking structure. The rotator is thereby rotated in the first rotational direction, releasing the first blocking structure from engagement with the second blocking structure. The delivery member cover is thereby able to move from the extended position towards the retracted position.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. An assembly for a medicament delivery device (1), the assembly comprising:
a body (3a),
a delivery member cover (9) arranged in the body (3a) and configured to move axially relative to the body (3a) from an extend position relative to the body (3a) to a retracted position,
a rotator configured to be rotatable relative to the body (3a), and
a cap (5) configured to receive a portion of the rotator when the delivery member cover (9) is in the extended position, and wherein the cap (5) is configured to be removably attached to and rotationally locked relative to the body (3a),
wherein the rotator has a first locking structure (9c) and the cap (5) has a second locking structure (5c) configured to engage with the first locking structure (9c) when the delivery member cover (9) is in the extended position, to prevent rotation of the rotator relative to the cap (5) in a first rotational direction and thereby maintain the rotator in a first rotational position relative to the body (3a),
wherein the delivery member cover (9) has a first blocking structure (9d) configured to engage with a second blocking structure (21), which is axially fixed relative to the body, when the rotator is in the first rotational position to prevent the delivery member cover (9) to move axially from the extended position towards the retracted position,
wherein one of the first blocking structure (9d) and the second blocking structure (21) has a cam surface (11a) configured to cooperate with the other one of the first blocking structure (9d) and the second blocking structure (21) such that when the cap (5) has been removed from the body (3a) and the delivery member cover (9) is subjected to an axial force directed distally, the rotator is rotated in the first rotational direction, causing the first blocking structure (9d) and the second blocking structure (21) to disengage, enabling the delivery member cover (9) to move axially from the extended position towards the retracted position;
wherein the delivery member cover (9) is the rotator, wherein the body (3a) comprises the second blocking structure (21), and wherein the cap (5) is configured to receive a proximal portion (9a) of the delivery member cover (9) when the delivery member (9) is in the extended position.

2. The assembly as claimed in claim 1, wherein one of the first locking structure (9c) and the second locking structure (5c) comprises a recess and the other one of the first locking structure (9c) and the second locking structure (5c) comprises a protrusion configured to be received into the recess to prevent rotation of the delivery member cover (9) in the first rotational direction.

3. The assembly as claimed in claim 2, wherein the recess is arranged in a radial plane of the delivery member cover (9).

4. The assembly as claimed in claim 2 or 3, wherein the recess extends in a circumferential direction of the delivery member cover (9).

5. The assembly as claimed in any of claims 2-4, wherein the protrusion extends in an axial direction of the cap (5).

6. The assembly as claimed in any of claims 2-5, wherein the first locking structure (9c) comprises the recess and the second locking structure comprises (5c) the protrusion.

7. The assembly as claimed in any of the preceding claims, wherein the first blocking structure (9d) comprises a first radial protrusion (11), and the second blocking structure (21) comprises a second radial protrusion configured to be arranged axially aligned with and distally relative to the first radial protrusion (11) in the first rotational position of the delivery member cover (9) to prevent the delivery member cover (9) to move towards the retracted position.

8. The assembly as claimed in claim 7, wherein the delivery member cover (9) comprises a radially extending first ramp structure (13) that tapers in a direction from the proximal portion towards a distal end of the delivery member cover (9), wherein the first ramp structure (13) is arranged offset in the first rotational direction relative to the first radial protrusion (11) and closer to the proximal portion (9a) than the first radial protrusion (11), and wherein the second radial protrusion is configured to move over the first ramp structure (13) when the delivery member cover (9) is moved towards the retracted position.

9. The assembly as claimed in claim 8, wherein the first ramp structure (13) has a proximal end portion (13a) provided with a radial guide surface (13b) extending along a helical direction of the delivery member cover (9), the radial guide surface (13b) being configured to guide the second radial protrusion when the delivery member cover (9) is moved towards the extended position, causing rotation of the delivery member cover (9) in a second rotational direction opposite to the first rotational direction relative to the body (3a).

10. The assembly as claimed in claim 9, wherein the delivery member cover (9) has a second ramp structure (17) which in the circumferential direction is arranged adjacent to the first ramp structure (13), wherein the radial guide surface (13b) is configured to guide the second radial protrusion towards the second ramp structure (17) when the delivery member cover (9) is moved from the retracted position towards the extended position, wherein the second ramp structure (17) is tapering in an opposite direction relative to the tapering of the first ramp structure (13).

11. The assembly as claimed in claim 10, wherein the second ramp structure (17) has a snap-lock functionality configured to prevent the delivery member cover (9) to move towards the retracted position when the second radial protrusion has moved axially past the second ramp structure (17).

12. The assembly as claimed in claim 11, wherein the delivery member cover (9) comprises a radial structure (18) axially aligned with and arranged distally relative to the second ramp structure (17), the radial structure (18) being configured to prevent the delivery member cover (9) to move further proximally when the second radial protrusion has moved past the second ramp structure (17).

13. A medicament delivery device (1) comprising an assembly as claimed in any of the preceding claims.

## Patentansprüche

1. Baugruppe für eine Medikamentenabgabevorrichtung (1), die Baugruppe umfassend:
einen Körper (3a),
eine Abgabeelementabdeckung (9), die in dem Körper (3a) angeordnet und konfiguriert ist, um sich relativ zu dem Körper (3a) von einer ausgefahrenen Position relativ zu dem Körper (3a) zu einer zurückgezogenen Position axial zu bewegen,
einen Rotator, der konfiguriert ist, um relativ zu dem Körper (3a) drehbar zu sein, und
eine Kappe (5), die konfiguriert ist, um einen Abschnitt des Rotators aufzunehmen, wenn die Abgabeelementabdeckung (9) in der ausgefahrenen Position ist, und wobei die Kappe (5) konfiguriert ist, um an dem Körper (3a) entfernbar angebracht zu werden und relativ zu dem Körper drehbar verriegelt zu werden,
wobei der Rotator eine erste Verriegelungsstruktur (9c) aufweist und die Kappe (5) eine zweite Verriegelungsstruktur (5c) aufweist, die konfiguriert ist, um mit der ersten Verriegelungsstruktur (9c) in Eingriff zu kommen, wenn die Abgabeelementabdeckung (9) in der ausgefahrenen Position ist, um eine Drehung des Rotators relativ zu der Kappe (5) in einer ersten Drehrichtung zu verhindern und dadurch den Rotator in einer ersten Drehposition relativ zu dem Körper (3a) zu halten,
wobei die Abgabeelementabdeckung (9) eine erste Blockierstruktur (9d) aufweist, die konfiguriert ist, um mit einer zweiten Blockierstruktur (21) in Eingriff zu kommen, die relativ zu dem Körper axial fixiert ist, wenn der Rotator in der ersten Drehposition ist, um zu verhindern, dass sich die Abgabeelementabdeckung (9) axial von der ausgefahrenen Position zu der zurückgezogenen Position hin bewegt,
wobei eine der ersten Blockierstruktur (9d) und der zweiten Blockierstruktur (21) eine Nockenoberfläche (11a) aufweist, die konfiguriert ist, um mit der anderen der ersten Blockierstruktur (9d) und der zweiten Blockierstruktur (21) zusammenzuwirken, derart, dass, wenn die Kappe (5) von dem Körper (3a) entfernt wurde und die Abgabeelementabdeckung (9) einer axial distal gerichteten Kraft ausgesetzt wird, der Rotator in der ersten Drehrichtung gedreht wird, wobei bewirkt wird, dass die erste Blockierstruktur (9d) und die zweite Blockierstruktur (21) außer Eingriff gebracht werden, wobei ermöglicht wird, dass sich die Abgabeelementabdeckung (9) axial von der ausgefahrenen Position zu der zurückgezogenen Position hin bewegt;
wobei die Abgabeelementabdeckung (9) der Rotator ist, wobei der Körper (3a) die zweite Blockierstruktur (21) umfasst, und wobei die Kappe (5) konfiguriert ist, um einen proximalen Abschnitt (9a) der Abgabeelementabdeckung (9) aufzunehmen, wenn das Abgabeelement (9) in der ausgefahrenen Position ist.

2. Baugruppe nach Anspruch 1, wobei eine der ersten Verriegelungsstruktur (9c) und der zweiten Verriegelungsstruktur (5c) eine Aussparung umfasst und die andere der ersten Verriegelungsstruktur (9c) und der zweiten Verriegelungsstruktur (5c) einen Vorsprung umfasst, der konfiguriert ist, um in die Aussparung aufgenommen zu werden, um eine Drehung der Abgabeelementabdeckung (9) in der ersten Drehrichtung zu verhindern.

3. Baugruppe nach Anspruch 2, wobei die Aussparung in einer radialen Ebene der Abgabeelementabdeckung (9) angeordnet ist.

4. Baugruppe nach Anspruch 2 oder 3, wobei sich die Aussparung in einer Umfangsrichtung der Abgabeelementabdeckung (9) erstreckt.

5. Baugruppe nach einem der Ansprüche 2 bis 4, wobei sich der Vorsprung in einer axialen Richtung der Kappe (5) erstreckt.

6. Baugruppe nach einem der Ansprüche 2 bis 5, wobei die erste Verriegelungsstruktur (9c) die Aussparung umfasst und die zweite Verriegelungsstruktur (5c) den Vorsprung umfasst.

7. Baugruppe nach einem der vorstehenden Ansprüche, wobei die erste Blockierstruktur (9d) einen ersten radialen Vorsprung (11) umfasst, und die zweite Blockierstruktur (21) einen zweiten radialen Vorsprung umfasst, der konfiguriert ist, um axial ausgerichtet mit und distal relativ zu dem ersten radialen Vorsprung (11) in der ersten Drehposition der Abgabeelementabdeckung (9) angeordnet zu sein, um zu verhindern, dass sich die Abgabeelementabdeckung (9) zu der zurückgezogenen Position hin bewegt.

8. Baugruppe nach Anspruch 7, wobei die Abgabeelementabdeckung (9) eine sich radial erstreckende erste Rampenstruktur (13) umfasst, die sich in einer Richtung von dem proximalen Abschnitt zu einem distalen Ende der Abgabeelementabdeckung (9) verjüngt, wobei die erste Rampenstruktur (13) in der ersten Drehrichtung relativ zu dem ersten radialen Vorsprung (11) versetzt und näher zu dem proximalen Abschnitt (9a) als der erste radiale Vorsprung (11) angeordnet ist, und wobei der zweite radiale Vorsprung konfiguriert ist, um sich über die erste Rampenstruktur (13) zu bewegen, wenn die Abgabeelementabdeckung (9) zu der zurückgezogenen Position hin bewegt wird.

9. Baugruppe nach Anspruch 8, wobei die erste Rampenstruktur (13) einen proximalen Endabschnitt (13a) aufweist, der mit einer radialen Führungsoberfläche (13b) bereitgestellt ist, die sich entlang einer spiralförmigen Richtung der Abgabeelementabdeckung (9) erstreckt, wobei die radiale Führungsoberfläche (13b) konfiguriert ist, um den zweiten radialen Vorsprung zu führen, wenn die Abgabeelementabdeckung (9) zu der ausgefahrenen Position hin bewegt wird, wobei eine Drehung der Abgabeelementabdeckung (9) in einer zweiten Drehrichtung entgegengesetzt zu der ersten Drehrichtung relativ zu dem Körper (3a) bewirkt wird.

10. Baugruppe nach Anspruch 9, wobei die Abgabeelementabdeckung (9) eine zweite Rampenstruktur (17) aufweist, die in der Umfangsrichtung zu der ersten Rampenstruktur (13) benachbart angeordnet ist, wobei die radiale Führungsoberfläche (13b) konfiguriert ist, um den zweiten radialen Vorsprung zu der zweiten Rampenstruktur (17) hin zu führen, wenn die Abgabeelementabdeckung (9) von der zurückgezogenen Position zu der ausgefahrenen Position hin bewegt wird, wobei sich die zweite Rampenstruktur (17) in einer entgegengesetzten Richtung relativ zu der Verjüngung der ersten Rampenstruktur (13) verjüngt.

11. Baugruppe nach Anspruch 10, wobei die zweite Rampenstruktur (17) eine Schnappverriegelungsfunktionalität aufweist, die konfiguriert ist, um zu verhindern, dass sich die Abgabeelementabdeckung (9) zu der zurückgezogenen Position hin bewegt, wenn sich der zweite radiale Vorsprung axial über die zweite Rampenstruktur (17) hinaus bewegt hat.

12. Baugruppe nach Anspruch 11, wobei die Abgabeelementabdeckung (9) eine radiale Struktur (18) umfasst, die mit der zweiten Rampenstruktur (17) axial ausgerichtet und relativ zu dieser distal angeordnet ist, wobei die radiale Struktur (18) konfiguriert ist, um zu verhindern, dass sich die Abgabeelementabdeckung (9) weiter proximal bewegt, wenn der zweite radiale Vorsprung über die zweite Rampenstruktur (17) hinaus bewegt wurde.

13. Medikamentenabgabevorrichtung (1), umfassend eine Baugruppe nach einem der vorstehenden Ansprüche.

## Revendications

1. Ensemble pour un dispositif d'administration de médicament (1), l'ensemble comprenant :
un corps (3a),
un couvercle d'élément d'administration (9) disposé dans le corps (3a) et conçu pour se déplacer de manière axiale par rapport au corps (3a) à partir d'une position déployée par rapport au corps (3a) vers une position rétractée,
un élément rotatif conçu pour pouvoir être rotatif par rapport au corps (3a), et
un capuchon (5) conçu pour recevoir une partie de l'élément rotatif lorsque le couvercle d'élément d'administration (9) se trouve dans la position déployée, et dans lequel le capuchon (5) est conçu pour être fixé de manière amovible et être verrouillé de manière rotative par rapport au corps (3a),
dans lequel l'élément rotatif a une première structure de verrouillage (9c) et le capuchon (5) a une seconde structure de verrouillage (5c) conçue pour venir en prise avec la première structure de verrouillage (9c) lorsque le couvercle d'élément d'administration (9) se trouve dans la position déployée, afin d'empêcher une rotation de l'élément rotatif par rapport au capuchon (5) dans une première direction de rotation et de maintenir ainsi l'élément rotatif dans une première position de rotation par rapport au corps (3a),
dans lequel le couvercle d'élément d'administration (9) a une première structure de blocage (9d) conçue pour venir en prise avec une seconde structure de blocage (21), qui est fixée de manière axiale par rapport au corps, lorsque l'élément rotatif se trouve dans la première position de rotation, afin d'empêcher le couvercle d'élément d'administration (9) de se déplacer de manière axiale à partir de la position déployée vers la position rétractée,
dans lequel l'une de la première structure de blocage (9d) et de la seconde structure de blocage (21) a une surface de came (11a) conçue pour coopérer avec l'autre de la première structure de blocage (9d) et de la seconde structure de blocage (21) de sorte que, lorsque le capuchon (5) a été retiré du corps (3a) et que le couvercle d'élément d'administration (9) est soumis à une force axiale dirigée de manière distale, l'élément rotatif tourne dans la première direction de rotation, ce qui amène la première structure de blocage (9d) et la seconde structure de blocage (21) à se libérer, et permet au couvercle d'élément d'administration (9) de se déplacer de manière axiale à partir de la position déployée vers la position rétractée ;
dans lequel le couvercle d'élément d'administration (9) est l'élément rotatif, dans lequel le corps (3a) comprend la seconde structure de blocage (21), et dans lequel le capuchon (5) est conçu pour recevoir une partie proximale (9a) du couvercle d'élément d'administration (9) lorsque l'élément d'administration (9) se trouve dans la position déployée.

2. Ensemble selon la revendication 1, dans lequel l'une de la première structure de verrouillage (9c) et de la seconde structure de verrouillage (5c) comprend un évidement et l'autre de la première structure de verrouillage (9c) et de la seconde structure de verrouillage (5c) comprend une saillie conçue pour être reçue dans l'évidement afin d'empêcher la rotation du couvercle d'élément d'administration (9) dans la première direction de rotation.

3. Ensemble selon la revendication 2, dans lequel l'évidement est disposé dans un plan radial du couvercle d'élément d'administration (9).

4. Ensemble selon la revendication 2 ou 3, dans lequel l'évidement s'étend dans une direction circonférentielle du couvercle d'élément d'administration (9).

5. Ensemble selon l'une quelconque des revendications 2 à 4, dans lequel la saillie s'étend dans une direction axiale du capuchon (5).

6. Ensemble selon l'une quelconque des revendications 2 à 5, dans lequel la première structure de verrouillage (9c) comprend l'évidement et la seconde structure de verrouillage (5c) comprend la saillie.

7. Ensemble selon l'une quelconque des revendications précédentes, dans lequel la première structure de blocage (9d) comprend une première saillie radiale (11), et la seconde structure de blocage (21) comprend une seconde saillie radiale conçue pour être disposée alignée de manière axiale sur et de manière distale par rapport à la première saillie radiale (11) dans la première position de rotation du couvercle d'élément d'administration (9) afin d'empêcher le couvercle d'élément d'administration (9) de se déplacer vers la position rétractée.

8. Ensemble selon la revendication 7, dans lequel le couvercle d'élément d'administration (9) comprend une première structure de rampe (13) s'étendant de manière radiale qui se rétrécit dans une direction à partir de la partie proximale vers une extrémité distale du couvercle d'élément d'administration (9), dans lequel la première structure de rampe (13) est disposée de manière décalée dans la première direction de rotation par rapport à la première saillie radiale (11) et plus proche de la partie proximale (9a) que la première saillie radiale (11), et dans lequel la seconde saillie radiale est conçue pour se déplacer sur la première structure de rampe (13) lorsque le couvercle d'élément d'administration (9) se déplace vers la position rétractée.

9. Ensemble selon la revendication 8, dans lequel la première structure de rampe (13) a une partie d'extrémité proximale (13a) pourvue d'une surface de guidage radiale (13b) s'étendant le long d'une direction hélicoïdale du couvercle d'élément d'administration (9), la surface de guidage radiale (13b) étant conçue pour guider la seconde saillie radiale lorsque le couvercle d'élément d'administration (9) se déplace vers la position déployée, provoquant la rotation du couvercle d'élément d'administration (9) dans une seconde direction de rotation opposée à la première direction de rotation par rapport au corps (3a).

10. Ensemble selon la revendication 9, dans lequel le couvercle d'élément d'administration (9) a une seconde structure de rampe (17) qui, dans la direction circonférentielle, est disposée à côté de la première structure de rampe (13), dans lequel la surface de guidage radiale (13b) est conçue pour guider la seconde saillie radiale vers la seconde structure de rampe (17) lorsque le couvercle d'élément d'administration (9) se déplace à partir de la position rétractée vers la position déployée, dans lequel la seconde structure de rampe (17) se rétrécit dans une direction opposée par rapport au rétrécissement de la première structure de rampe (13).

11. Ensemble selon la revendication 10, dans lequel la seconde structure de rampe (17) a une fonction de verrouillage par encliquetage conçue pour empêcher le couvercle d'élément d'administration (9) de se déplacer vers la position rétractée lorsque la seconde saillie radiale s'est déplacée de manière axiale au-delà de la seconde structure de rampe (17).

12. Ensemble selon la revendication 11, dans lequel le couvercle d'élément d'administration (9) comprend une structure radiale (18) alignée de manière axiale sur et disposée de manière distale par rapport à la seconde structure de rampe (17), la structure radiale (18) étant conçue pour empêcher le couvercle d'élément d'administration (9) de se déplacer davantage de manière proximale lorsque la seconde saillie radiale s'est déplacée au-delà de la seconde structure de rampe (17).

13. Dispositif d'administration de médicament (1) comprenant un ensemble selon l'une quelconque des revendications précédentes.
